# EUROPEAN PATENT APPLICATION

(11) **EP 4 520 319 A1**
(43) Date of publication of application: **12.03.2025**
(21) Application number: 23724335.7
(22) Date of filing: 19.04.2023
(51) Int. Cl.: A61K 9/00, A61K 47/02, A61K 47/26, A61K 47/36

(54) **PRODUCT FOR ENHANCING FERTILITY**

(30) Priority: 04.05.2022 EP 22382428
(71) Applicant: Ecareyou Innovation, S.L., 08173 Sant Cugat del Vallès (ES)
(72) Inventor: GARRIGA I RODO, Joan, 08173 Sant Cugat Del Valles (Barcelona) (ES)
(74) Representative: Espiell Gomez, Ignacio
(86) International application number: PCT/ES2023/070247
(87) International publication number: WO 2023/214101

(57) **Abstract**

Product to enhance the fertility , consisting in a gel for vaginal topic use, using a physical device (plug) in a post-coital form, that comprises: an aqueous base; calcium chosen among calcium bicarbonate of calcium chloride; myo-inositol; a prebiotic, chosen among FOS or alpha-GOS; sodium phosphate, sodium hydroxide or potassium phosphate, to adjust the Ph of the solution between 7 to 8.5; sodium chloride to maintain the osmolarity between 280 and 300 mOsm/l; low methylation pectin, hydroxypropylmethyl cellulose, carrageenan or polyacrylic acid to maintain the viscosity between 1.5 and 5 cP at 37°C; hyaluronic acid and potassium sorbate, chlorphenesin or phenoxyethanol as preserving agent.

## Description

### PRODUCT TO ENHANCE THE FERTILITY

### OBJECT OF THE INVENTION

The invention, as the title of the specification sets, refers to a product to enhance the fertility.

The object of this invention falls on a product that consists in a composition in the form of gel for vaginal topic use that, devised to be used jointly with a physic device (plug) in postcoital form, has the objective of improving the short- and long-term fertility by means of different synergic action mechanisms and not competitive to each other. Concretely, the said product is based in a formulation with a base of water that, among other components, essentially comprises a calcium component, that provokes the activation in the sperm, a prebiotic component that prevents inappropriate conditions for the sperm in the vagina, and a certain amount of myoinositol that reduces the number of non-motile spermatozoids.

### FIELD OF APPLICATION OF THE INVENTION

The field of application of this invention is within the sector of the chemical industry, particularly focusing on the field of the industry engaged in formulation of medical and/or pharmaceutical products, concretely focused in the topic use products designed to enhance the fertility.

### BACKGROUND OF THE INVENTION

As reference to the current state-of-the-art. It shall be pointed out that, at least the applicant has not identified products having an identical use of an identical or similar formulation. Therefore, it can be understood that, currently, no identical technical solutions exist.

The Product and the associated device have the aim of offering to the market a technical alternative and/or an additional support to the usual practice of raising the legs after the coitus to prevent that the sperm gets out.

However, products are actually localized the application, formulation and/or operating instructions of which are similar, to wit:
- Vaginal lubricants in general and sperm-friendly (also known in the market as *sperm-friendly or fertility-friendly lubricants)* vaginal lubricants namely: these products have as objective to physically improve the lubrication during the sexual intercourse and have not as objective nor use to contain any ingredient that, actively, enhances or improves the fertility nor has a positive influence in any other aspect of the fertility in addition to the physical coitus process. Their use is pre-coitus and their objective is closer from the leisure than from medicine.

The term sperm-friendly is self-applied because of the purported lack, in its formulation, of elements or ingredients that could be aggressive to the sperm, such as parabens and glycerol. These ingredients fulfil the task of preserving the product (parabens) or a physical function/ matrix (glycerol) and their presence can result toxic for the spermatozoids. Even products self-named sperm-friendly use to contain these potentially deleterious active ingredients. However, a minority actually meet the chemical premises necessary to be deemed sperm-friendly.

On the other hand, although some of the said products contain ingredients that are coincident with the formulation of the product of this invention, such as calcium and prebiotics such as arabinogalactan and xylose, the presence of fructo-oligosaccharides (FOS) or alpha-Glucan-oligosaccharides (alpha-GOS) has not been identified in this type of products.
- Vagina moisturizers/lubricants: there exists a great variety of this type of products, that have as objective to provide moisture and lubrication to the vagina of people having vaginal dryness. Therefore, the population to whom this product is addressed and its objective are different to those of the herein proposed product. In addition, and regardless that they can be used in another manner, in principle, they are designed to a pre-coitus use or independent from the coitus. They have not as objective to enhance the fertility nor to provoke any change in the sperm, nor to promote the fecundation. Their mechanism of action is solely physical instead of biological or biochemical.

In the said products the lack of parabens is frequent (for business more than technical reasons or to preserve the sperm) and also the presence of hyaluronic acid as lubricant.

In some products of this category in the Spanish to international market, they possess prebiotics of the alpha-GOS type, but the use of FOS was not identified in them. The presence of prebiotic in these products increases the range of their mechanism of action and extends it to an also biochemical one, solely at a level of vaginal microbiota and not at a spermatic level.
- Vaginal prebiotics: there also exists a certain number of products the objective of which it to protect or improve the vaginal flora/ microbiota by providing substrates of growth that can be used by the more present microorganism in the vagina (Lactobacillus type), named prebiotics.

These products have a use independent from the coitus and their objective is to prevent or revert bacterial, fungal or viral infections in the vagina, but they are not designed to improve or promote the fertility or the fecundation. Their mechanism of action is biochemical solely on the microorganisms of the vagina, without affecting the sperm or the spermatozoids, with which, given their mode of use, they should not have to contact.

In the Spanish market, different vaginal prebiotic products have been identified, the objective of which is to prevent or treat the vaginal injuries caused by the human papillomavirus or they are products of personal hygiene specific for the vagina but with a more general use.

Last, it shall be mentioned that, in the scientifical literature, publications have been found in which some researchers had tested the usefulness of a prebiotic, a fructo-oligosaccharide type (FOS), concretely the Actilight^{®} of the company Tereos, in vaginal applications.

However, it has to be pointed out that in these publications the material used is simply Actilight^{®} FOS, without any galenic formulation or accompanying ingredients and that the objective of the experiment and, therefore, the product, was to promote beneficial microorganisms and not to promote pathogens and that it does not mention fecundation at all. The mechanism of action of the ingredient is exclusively biochemical and exclusively on the vaginal microbiota (in fact, on three strains of three very concrete species, that do not faithfully represent the real complexity of the vaginal microbiota).

### EXPLANATION OF THE INVENTION

The product to enhance the fertility that the invention proposes, as it was said before, is a product that has as aim to improve the fertility at short and long term. This is achieved by means of the following mechanisms:
- Generating an optimum environment (moisture, lubrication, pH, viscosity) in the vagina in order that the changes in the sperm and the spermatozoids necessary for the fecundation can occur.
- Biochemically promoting these changes in the spermatozoids by means of their chemical composition and physical-chemical characteristics (mainly in process of spermatic capacitation).
- Accompanying / dragging the sperm inwards the vagina to reach the uterus, without hindering this process because of its low viscosity and protecting the spermatozoids from physical-chemical aggressions (pH, osmolarity).
- Feeding the beneficial vaginal flora (Lactobacillus) to prevent bacterial infections and changes of pH. A healthy vaginal microbiota is linked to a best fertility.

The presentation of the product is a gel for vaginal topic use after the coitus (postcoital use), matched with a physical plug-like device, which does not form part of the object of this invention, because it can be a device of those already existing in the market, which serves to retaining the gel within the vagina and prevent it gets out, extending its presence within it.

Thus, the product contacts as well the vaginal fluids as the sperm, with which it mixes.

Its formulation, as well at the level of presence of active ingredients, as of the absence of certain ingredients, as of physical-chemical characteristics, is optimized to meet the objective as well at physical level as at chemical level, as biological/biochemical level.

As for the physical-chemical characteristics, the product has an aqueous base (about 96% of purified water) to emulate the composition of the cervical mucus in the preovulatory period, when the fertility is maximum. The seminal liquid has a similar aqueous composition.

Its pH ranges between 7 and 8.5 values. The vagina has a slightly more acid pH than these values, but during the coitus its pH rises up to 7 to improve the viability of the sperm, by means of generating a vaginal discharge. On its part, the seminal liquid possesses a higher pH to overcome the vagina basal acidity, of up to 8.5. Therefore, the pH of the product has as objective to protect the sperm. Different ingredients exist with which the pH of the solution can be adjusted and, in principle, the choice of one or another is not critical for the operation of the product, therefore the presence of one of them in particular can be deemed as not critical. The sodium phosphate, the sodium hydroxide and the potassium phosphate are suggested.

The osmolarity is crucial to maintain the viability of the sperm because a hypoosmotic medium would cause the explosion of the spermatozoids and a hyperosmotic medium, their desiccation. The product has an osmolarity ranging from 280 to 300 mOsm/l emulating that of the vaginal fluids and that of the sperm. Preferably, this osmotic pressure is reached by adding sodium chloride, although any other ingredient could be used (the sodium chloride can be in addition deemed not critical although the maintenance of the osmolarity in this range actually is).

The viscosity of one of the most important factors at physical level, because an excessive viscosity in the medium could make the spermatozoids swimming/advancing difficult and a too low viscosity could physically drag them outwards the vagina by gravity (even despite the presence of the plug). A viscosity ranging from 1.5 to 5 cP at 37°C is proposed, reached, preferably, with low methylation pectin in an approximate proportion of 3% weight/volume and polymerized with the assistance of calcium. The need to add calcium to achieve a technical aim is clinically beneficial, as it was explained before and will be detailed again thereafter. Optionally, the pectin can be replaced by other ingredients that allow to reach the expected viscosity, such as the hydroxipropylmethylcellulose (also known as hypromellose), the carrageenan or the polyacrylic acid.

To obtain a suitable moisture and lubrication in the vagina, it is proposed to use of hyaluronic acid in an approximate proportion of 0.5% in weight/volume. The presence of the hyaluronic acid and in these proportions has been identified in different products in the market.

The product meets its objective, in addition to by physical chemical mediums, by biological/biochemical mediums for which the following active ingredients are essential:
- Calcium, concretely in the form of calcium bicarbonate or of calcium chloride, the concrete form is not critical. In addition to fulfil a technical function in the product, as it was already commented, the calcium plays a fundamental role in the process of spermatic capacitation, that consists in a series of biochemical changes that occur in the sperm when this later is located in the vagina after the ejaculation and that allow its activation to complete the fecundation of the egg. Therefore, and regardless the presence of pectins as gelling agent, the presence of calcium is crucial and in addition it means a very clear differentiating element against other products, that do not use to give attention to the spermatic capacitation.
- Myo-inositol: the myo-inositol, according to studies carried out, is a compound of inositol that, in contact with the sperm reduces the number of non-motile spermatozoids and in contact with the cervical mucus improves its viscosity for the fecundation.
- Prebiotic, preferably in form of FOS (fructo-oligosaccharides), as commercialized by Actilight^{®} or other, or in form of alpha-GOS (oligosaccharide of alpha-glucan). The vaginal microbiota plays an important role in the fertility. The presence of pathogen microorganisms in the vagina can generate inappropriate biological, physical and chemical conditions for the sperm, which can affect its capacity to fertilize the egg.

In addition, it is important to point out that the product has to be parabens- and glycerin-free, to preserve the viability of the sperm. As preserving agents potassium sorbate or others can be used.

Summarizing, therefore, the product to enhance the fertility, object of this invention, being formed by composition in the form of gel, essentially comprises, at least, the following:
- an aqueous base, preferably purified water;
- a component of calcium, chosen among calcium bicarbonate or calcium chloride;
- a component of inositol, concretely myo-inositol; and
- a prebiotic component, chosen among FOS (fructo-oligosaccharides) or alpha-GOS (oligo-saccharide of alpha-glucan).

However, in addition to the said components, the product preferably also comprises:
- an alkalinization component, to adjust the Ph of the solution between 7 and 8.5, preferably sodium phosphate or sodium hydroxide or potassium phosphate;
- a component that maintains the osmolarity between 280 and 300 mOsm/l, preferably sodium chloride; and
- a component that maintains the viscosity between 1.5 and 5 cP at 37°C, preferably low methylation pectin or hydroxypropylmethylcellulose, carrageenan or polyacrylic acid;

In addition, in an embodiment, the product likewise comprises:
- hyaluronic acid, to optimize the moisture and lubrication; and
- a preserving component: preferably potassium sorbate or chlorphenesine of phenoxyethanol.

With it, the example of preferred embodiment of the product will comprise the following ingredients in the proportions indicated:
- 93% purified water;
- calcium chloride;
- myo-inositol;
- FOS (fructo-oligosaccharides) at 5% (w/v);
- sodium phosphate;
- sodium chloride;
- carrageenan 0.1% (w/v);
- hyaluronic acid 0.5% (w/v);
   and
- potassium sorbate.

With all that, the main differentiating characteristics and advantages of the product object of the invention against others are:
- Its objective and application: the similar products do not have as objective to actively improve the fertility or the process of fecundation. Their aims are, at most, not to hinder it by means of the absence of toxic substances for the spermatozoids. The possibility to add certain assets to the sperm to improve its effectiveness in the fecundation at short or long term, is not obvious.
- Mechanisms of action mainly focused in the sperm and the spermatozoids, instead of in the sexual intercourse and/or the vagina. Most of the similar products base their effectiveness solely in the optimization of the conditions of vagina (lubrication, moisture, microbiota). However, no products designed to be mixed with the sperm and improve its effectiveness at physical, chemical and biological level have been identified.
- Mechanisms of multiple actions, synergetic and noncompetitive to enhance the fertility as well at short as at long term:
   - Physical: accompanying and dragging the sperm towards the uterus, with the optimum viscosity and lubrication, replicating the conditions of the sperm fluid.
   - Chemical: protecting pH and osmolarity of the spermatozoids, replicating the conditions of the sperm fluid.
   - Biological-human: promoting the process of sperm capacitation, sperm motility and production of high-quality cervical mucus.
   - Microbiological: promotion of healthy vaginal flora, resistant to infection and pathogen proliferation.
- Mode of use: the product has a postcoital application to meet its aim, instead of pre-coital or independent from the coitus, as it occurs with other products.
- Composition: the proposed formula is original, not present in any other product.

Sufficiently disclosed the nature of this invention, as well as the manner of implementing it, it is not deemed necessary to extend its explanation in order that a person skilled in the art understands its extent and the advantages arising from it.

## Claims

1. - Product consisting on a composition that comprises, at least:
- an aqueous base;
- a component of calcium, chosen among calcium bicarbonate or calcium chloride;
- myo-inositol; and
- a prebiotic component, chosen among FOS (fructo-oligosaccharides) or alpha-GOS (oligo-saccharide of alpha-glucan),
for use to enhance the fertility, wherein the composition is in form of a gel for vaginal topic use, devised to be used jointly with a physical device (plug) in post-coital form.

2. The product for use to enhance the fertility, according to the claim 1, that comprises, in addition:
- an alkalinization component, to adjust the Ph of the solution from 7 to 8.5, chosen among sodium phosphate or sodium hydroxide or potassium phosphate;
- sodium chloride as component that maintains the osmolarity between 280 and 300 mOsm/l; and
- a component that maintains the viscosity between 0.015 P and 0.05 P (1.5 and 5 cP) at 37°C, preferably low methylation pectin or hydroxypropylmethylcellulose, carrageenan or polyacrylic acid;

3. The product for use to enhance the fertility, according to the claim 1 or 2, that comprises in addition:
- hyaluronic acid, and
- a preserving component: preferably potassium sorbate or chlorphenesin or phenoxyethanol.

4. The product for use to enhance the fertility, according to the claim 1, that comprises, as aqueous base, purified water.

5. The product for use to enhance the fertility, according to any of the preceding claims, that comprises:
- 93% purified water;
- calcium chloride;
- myo-inositol;
- sodium phosphate;
- sodium chloride;
- carrageenan 0.1% (w/v);
- hyaluronic acid 0.5% (w/v); and
- potassium sorbate.
